# EUROPÄISCHE PATENTANMELDUNG

(11) **EP 4 644 522 A1**
(43) Veröffentlichungstag der Anmeldung: **05.11.2025**
(21) Anmeldenummer: 24172987.0
(22) Anmeldetag: 29.04.2024
(51) Int. Cl.: C12M 1/00, B01B 1/00, C12M 1/34

(54) **VERDAMPFERVORRICHTUNG FÜR EINEN INKUBATOR, INKUBATOR UND VERFAHREN**

(71) Anmelder: Eppendorf SE, 22339 Hamburg (DE)
(72) Erfinder: Fitzer, Jan, Ammersbek (DE); Maltzen, Hauke, Itzstedt (DE); Schünemann, Patrick, Hamburg (DE)
(74) Vertreter: Wallinger Ricker Schlotter Tostmann

(57) **Zusammenfassung**

Die Erfindung betrifft eine Verdampfervorrichtung und ein Verfahren zum Erzeugen eines Wasserdampfes zur Befeuchtung einer Inkubatoratmosphäre mittels einer Heizfläche der Verdampfervorrichtung, wobei Wasser mittels einer Wasserführungseinrichtung an der Wasserführungseinrichtung entlang fließend bis zu der Heizfläche geführt wird, die das Wasser verdampft.

## Beschreibung

Die Erfindung betrifft eine Verdampfervorrichtung für ein Laborgerät mit Inkubationsfunktion, insbesondere einen Inkubator für das Wachstum von Mikroorganismen, insbesondere von Zellen. Die Erfindung betrifft zudem ein Laborgerät mit dieser Verdampfervorrichtung und ein Verfahren zur Befeuchtung einer Inkubatoratmosphäre.

Mit solchen inkubationsfähigen Laborgeräten, zum Beispiel Inkubatoren oder inkubierten Shakern, werden in biologischen und medizinischen Laboratorien Proben unter kontrollierten Umgebungsbedingungen gehalten. Temperatur und Gaszusammensetzung bzw. die Luftfeuchtigkeit der Atmosphäre im Inneren einer von der Umgebung isolierten Inkubatorkammer werden durch die apparativen Einrichtungen des Inkubators auf den gewünschten Werten gehalten. Die Kontrolle der Luftfeuchtigkeit im Inkubator ist entscheidend, da sie das Wachstum und die Entwicklung von Embryonen, Zellen oder Mikroorganismen beeinflusst. Unterschiedliche Arten von Organismen erfordern unterschiedliche Luftfeuchtigkeitsniveaus, daher ist es wichtig, die Feuchtigkeit möglichst präzise zu steuern, um optimale Bedingungen für die Inkubation zu gewährleisten. Inkubatoren für Zellen in Zellkultur ermöglichen das Wachstum lebender Zellen in vitro unter definierten atmosphärischen Bedingungen. Eukaryotische Zellen benötigen COz-Inkubatoren. Die Atmosphäre wird durch Luft mit einem bestimmten CO₂- und Oz-Gehalt und einer bestimmten Luftfeuchtigkeit gebildet, eine geeignete Temperatur ist oftmals 37 °C.

Der Innenraum eines Inkubators oder inkubierten Shakers muss mit einer hohen Luftfeuchte geregelt werden. Typisch sind relativ Luftfeuchtewerte um die 85- 95%. Das kann zum einen passiv gelöst werden, indem eine offene Wasserfläche innerhalb des inkubierten Raumes temperiert wird oder durch eine aktive Regelung von verdampftem Wasser, das in die Kammer gebracht wird. Eine hohe und reproduzierbare Luftfeuchte ist wichtig, um zu verhindern, dass Medium aus inkubierten Zellkulturen verdunsten kann und somit Bestandteile aufkonzentriert werden. Reproduzierbarkeit ist eine Kernanforderung von Experimenten in der Zellkultur, so dass Bedingungen innerhalb des inkubierten Raumes immer reproduzierbar geregelt werden müssen. Ein weiterer wichtiger Aspekt ist die Gewährleistung von kondensatfreien Flächen im inkubierten Raum, um Kontaminationsbildung an diesen Stellen zu vermeiden.

Die passive Befeuchtung durch offene Wasserflächen in inkubierten Geräten hat den gro-βen applikativen Nachteil, dass offene Wasserflächen eine hohe Gefahr der Kontaminationsbildung in sich tragen - insbesondere von Pilzen. Schwappgefahr beim Entnehmen von Wasserwannen ist hierbei ebenfalls ein Problem, genauso wie der hohe Platzbedarf innerhalb des inkubierten Raumes, der nicht sinnvoll genutzt werden kann. Zudem ist diese Regelung passiv und kann nicht auf Sollwerte eingestellt werden. Bei Verdampfersystemen, die aktiv geregelt werden, ergibt sich die Herausforderung, dass das zu verdampfende Wasser reproduzierbar in sehr kleinen Dosen verdampft werden muss, um kein unverdampftes Wasser in den inkubierten Raum einzubringen, was zu offenem Wasser auf Oberflächen führt.

Bekannte Verdampfer lassen Wasser insbesondere auf die Oberfläche der Heizeinrichtung abtropfen, wie dies beispielsweise in DE 10 2005 030822 A1 vorgesehen ist. Diese Anordnungen bergen, wie in dieser Erfindung zugrunde liegenden Experimenten festgestellt wurde, den Nachteil, dass Dampf in unkontrollierter Weise erzeugt wird, da bei heißen Flächentemperaturen der Leidenfrost-Effekt auftreten kann. Wenn die Temperatur an der Grenzfläche ausreichend hoch ist, um eine schnelle primäre Verdampfung zu ermöglichen, bewegt sich der Wassertropfen auf einem Dampfpolster, das ihn vor direktem Wärmeaustausch schützt. Dieses Dampfpolster bildet sich unter dem Tropfen und entweicht nur langsam. Gleichzeitig bildet sich kontinuierlich neuer Wasserdampf, wodurch der Tropfen scheinbar über der heißen Oberfläche gleitet. Da Tropfen nie ganz symmetrisch sind, bilden sich zufällige Tropfenbewegungen auf der Oberfläche, die zu einer unkontrollierten Verdampfung führen. Solche Anordnungen sind deshalb für eine präzise Luftfeuchteregelung nicht optimal.

Der Erfindung liegt die Aufgabe zugrunde, eine Verdampfervorrichtung für ein Laborgerät mit Inkubationsfunktion bereitzustellen, die ein kontrolliertes Verdampfen ermöglicht.

Die Erfindung löst die Aufgabe durch die Verdampfervorrichtung gemäß Patentanspruch 1, das Laborgerät gemäß Patentanspruch 14 und das Verfahren gemäß Patentanspruch 15. Bevorzugte Ausgestaltungen dieser Gegenstände werden in den abhängigen Ansprüchen angegeben und lassen sich ferner der Beschreibung der Erfindung sowie den Figuren entnehmen.

Die Verdampfervorrichtung ist vorzugsweise ein Bestandteil eines Laborgeräts. Sie ist insbesondere eingerichtet zur Verbindung mit der Inkubationskammer eines Laborgerätes, das der Inkubation von Mikroorganismen, insbesondere lebenden Zellen, dient. Die Regelung der Luftfeuchte in der Kammer erfolgt bei einer solchen Verdampfervorrichtung aktiv, indem bedarfsweise Dampf in dosierter Weise erzeugt und der Kammer zugeführt wird. Die für ein kontrolliertes Verdampfen notwendige präzise Wasserdosierung wird gemäß der Erfindung erreicht, indem die Verdampfervorrichtung eine Wasserführungseinrichtung aufweist, durch die das von der Wassereintrittsöffnung in die Verdampferkammer eingetretene Wasser bis zu der Heizfläche an der Wasserführungseinrichtung entlang fließend führbar ist. Insbesondere wird durch die Wasserführungseinrichtung das Wasser bis an einen Rand der Heizfläche geführt. Diese Anordnung bewirkt, dass das Fallen von Tropfen auf die Heizfläche vermieden wird und somit insbesondere eine zeitlich nicht mehr präzise kontrollierbare Wasserdampffreisetzung vermieden wird.

Vorzugsweise ist die Wasserführungseinrichtung so ausgebildet, dass das durch die Wassereintrittsöffnung beförderte Wasser auf die Wasserführungsfläche fließt und/oder auf der Wasserführungsfläche fließt, insbesondere in Form eines Wasserfilms auf der Wasserführungsfläche, insbesondere ohne dass ein freier Wassertropfen auf die Heizfläche fällt.

Vorzugsweise weist die Wasserführungseinrichtung eine Wasserführungsfläche auf, die vorzugsweise zwischen der Wassereintrittsöffnung und der Heizfläche angeordnet ist. Vorzugsweise ist die Wasserführungsfläche im Kontakt mit der Heizfläche angeordnet. Es kann aber auch ein geringer Abstand zwischen Wasserführungsfläche und Heizfläche bestehen, insofern das Wasser fließend bis zur Heizfläche führbar ist, insbesondere ohne dass das Wasser auf die Heizfläche fällt. Die Wasserführungsfläche ist vorzugsweise in einem thermischen Kontakt mit der Heizfläche angeordnet. Die Wasserführungsfläche beginnt vorzugsweise an der Wassereintrittsöffnung. Die Wasserführungsfläche steht vorzugsweise mit der Mündung der Wassereintrittsöffnung in Kontakt. Die Wasserführungsfläche ist vorzugsweise planar. Die Wasserführungsfläche kann auch abschnittsweise gekrümmt verlaufen. Die Wasserführungsfläche ist - bei bestimmungsgemäßer Anordnung der Verdampfervorrichtung in montierter Position - vorzugsweise vertikal angeordnet.

Die Wasserführungsfläche kann so ausgestaltet sein, insbesondere hydrophil durch geeignete Oberflächenstruktur und/oder Materialwahl oder durch eine Beschichtung mit geeignetem Material, so dass das an der Wasserführungsfläche entlang fließende Wasser die Form eines Wasserfilms hat, oder dass der Wassertropfen auf der Wasserführungsfläche einen Kontaktwinkel zeigt, der kleiner ist als 90 Grad oder 60 Grad oder 45 Grad.

Die Position der Wassereintrittsöffnung ist derart, dass das in die Kammer eintretende Wasser mittels der Wasserführungseinrichtung bis zur Heizfläche führbar ist. Dabei kann die Wassereintrittsöffnung in Bezug auf die Gravitation oberhalb, neben oder sogar unterhalb der Heizfläche angeordnet sein.

Der Öffnungsquerschnitt der Wassereintrittsöffnung ist weist vorzugsweise einen maximalen Durchmesser auf, der zwischen 0,5 mm und 20 mm beträgt, vorzugsweise zwischen 1 mm und 5 mm, vorzugsweise zwischen 2 mm und 4 mm. Insbesondere durch diese geringen Durchmesser lässt sich eine feine Dosierung des in die Verdampferkammer zugeführten Wassers erreichen.

Die Wasserführungsfläche erfüllt ihren Zweck als wasserführendes Element einerseits in dem Fall, dass ein gravitationsbedingter Wasserfluss entlang der Wasserführungsfläche erfolgt. Im Betrieb wird die Verdampfervorrichtung insbesondere so montiert, dass sich die Heizfläche in Bezug auf Gravitationsrichtung unterhalb der Wassereintrittsöffnung befindet. Eine kleines, von der Wasserfördereinrichtung durch die Wassereintrittsöffnung zugeführtes Wasservolumen wird dann an der Wasserführungsfläche entlang fließen, ohne im freien Fall auf die Heizfläche aufzutreffen. Dabei ist es möglich, dass das Wasservolumen auf die Wasserführungsfläche fällt, solange das direkte Auftreffen auf der Heizfläche vermieden wird.

Vorzugsweise weist die Verdampferkammer einen Wandabschnitt, insbesondere Innenwandabschnitt, auf, der diese Wasserführungsfläche aufweist oder bildet. Der Innenwandabschnitt verläuft vorzugsweise ausgehend von der Wassereintrittsöffnung bis zur Heizfläche.

Die Wasserführungsfläche verläuft vorzugsweise nicht horizontal und verläuft vorzugsweise vertikal, wenn die Verdampfervorrichtung im bestimmungsgemäßen Gebrauch an der Wand einer Inkubationskammer eines Laborgerätes montiert ist. Die Wasserführungsfläche verläuft vorzugsweise nicht horizontal und verläuft vorzugsweise in einem Winkel α zur Horizontalen, wenn die Verdampfervorrichtung im bestimmungsgemäßen Gebrauch an der Wand einer Inkubationskammer eines Laborgerätes montiert ist, so dass Wasser gravitationsgetrieben an der Wasserführungsfläche entlang fließen kann, insbesondere so, dass kein Tropfen auf die Heizfläche fällt. Vorzugsweise 80° <= α <= 175°.

Im Betrieb wird die Verdampfervorrichtung insbesondere so montiert, dass sich die Heizfläche in Bezug auf Gravitationsrichtung unterhalb der Wassereintrittsöffnung befindet. Ein kleines, von der Wasserfördereinrichtung durch die Wassereintrittsöffnung zugeführtes Wasservolumen wird dann an der Wasserführungsfläche entlang fließen, ohne im freien Fall auf die Heizfläche aufzutreffen. Dabei ist es möglich, dass das Wasservolumen auf die Wasserführungsfläche fällt, solange das direkte Auftreffen auf der Heizfläche vermieden wird.

Die Wasserführungsfläche erfüllt ihren Zweck als wasserführendes Element auch in dem Fall, dass kein gravitationsbedingter Wasserfluss entlang der Wasserführungsfläche erfolgt. Es kann insbesondere vorgesehen sein, dass die Wasserführungsfläche dazu angeordnet ist, dass das von der Wassereintrittsöffnung in die Kammer beförderte Wasser mithilfe der Wasserfördereinrichtung über die Wasserführungsfläche weiter zur Heizfläche befördert wird. Die dazu erforderliche Förderenergie bzw. elektrische Leistung kann von der Wasserfördereinrichtung geliefert werden, so dass auf einen gravitationsgetriebenen Fluss verzichtet werden kann. Beispielsweise kann die Wasserführungsfläche horizontal verlaufen, insbesondere zwischen der Wassereintrittsöffnung und der Heizfläche. Solange das Wasser bis zur Heizfläche förderbar ist, kann die Wasserführungsfläche ein Wasservolumen auch nach oben leiten, angetrieben insbesondere durch die Wasserfördereinrichtung oder durch Kapillarkräfte. Insbesondere kann die Wasserführungsfläche die Innenfläche einer rohrförmigen Struktur sein, insbesondere eines Rohres oder Schlauches, das/der insbesondere von der Wassereintrittsöffnung bis zur Heizfläche verlaufen kann.

Die Dampfaustrittsöffnung ist vorzugsweise oberhalb der Heizfläche angeordnet, wenn die Verdampfervorrichtung im bestimmungsgemäßen Gebrauch an der Wand einer Inkubationskammer eines Laborgerätes montiert ist. Das Laborgerät wird im bestimmungsgemäßen Gebrauch auf einer horizontalen Ebene aufgestellt, so dass die Orientierung der Verdampfervorrichtung in Relation zum Laborgerät erfolgt. Diese Orientierung ist insofern zwingend, da flüssige Proben in Inkubationskammern in der Regel in offenen Gefäßen gelagert sind, die eine Gasaustausch zwischen Gefäßinnerem und der Inkubatoratmosphäre ermöglichen. Kulturgefäße für Mikroorganismen, insbesondere Zellen, sind gewöhnlich für eine Lagerung auf horizontalen Flächen konzipiert.

Vorzugsweise weist die Verdampfervorrichtung eine Wasserrückführungseinrichtung auf, durch die insbesondere ein im Bereich zwischen der Dampfaustrittsöffnung und der Heizfläche auftretendes Kondenswasser bis zu der Heizfläche an der Wasserrückführungseinrichtung entlang fließend rückführbar ist. Durch die Wasserrückführungseinrichtung wird vermieden, dass Kondenswasser sich nahe der Dampfaustrittsöffnung in der Verdampfervorrichtung oder sich sogar in der Dampfaustrittsöffnung sammelt. Druckschwankungen in der Verdampferkammer können mitunter dazu führen, dass Wassermengen nahe der Dampfaustrittsöffnung unvorhersehbar in Richtung einer mit der Dampfaustrittsöffnung verbundenen Inkubationskammer ausgestoßen werden. Zudem kann ein unkontrollierter Rückfluss des Wassers zu unkontrollierter Verdampfung führen, wenn Kondensat unterschiedlicher Menge in einem unvorhersehbaren zeitlichen Verlauf auf die Heizfläche trifft und erneut verdampft wird.

Vorzugsweise ist die Wasserrückführungseinrichtung in thermischem Kontakt stehend mit der Heizfläche angeordnet. Da die Wasserrückführungseinrichtung in diesem Fall stetig erwärmt bleibt, wird die Wasserrückführung gegenüber nicht-erwärmten Wasserrückführungseinrichtungen noch besser kontrollierbar, wie in Experimenten festgestellt wurde.

Vorzugsweise weist die Verdampfervorrichtung einen Dampfauslasskanal auf, der sich zwischen der Verdampferkammer und der Dampfaustrittsöffnung entlang einer Längsachse erstreckt. Der Dampfauslasskanal kann zylinderförmig sein. Der Dampfauslasskanal kann in Richtung der Dampfaustrittsöffnung durch eine Stirnwand verschlossen sein, welche insbesondere diese Dampfaustrittsöffnung aufweist. Die Stirnwand ist vorzugsweise im Wesentlichen planar und parallel zu einer Ebene E ausrichtbar. Die Stirnwand ist vorzugsweise dazu eingerichtet, parallel zur Seitenwand einer Inkubationskammer angeordnet zu sein und/oder insbesondere mit der Seitenwand verbindbar zu sein. Die Längsachse verläuft vorzugsweise in einem Winkel β zu der Normalen der Ebene E, wobei vorzugsweise 0 < β <= 90 Grad, vorzugsweise 2 <= β <= 60 Grad, vorzugsweise 3 <= β <= 60 Grad. Durch diese Neigung des Dampfauslasskanals gegenüber der Horizontalen fließt das Kondenswasser gravitationsbedingt kontrolliert zurück zur Heizfläche.

Vorzugsweise weist die Wasserrückführungseinrichtung eine Wasserrückführungsfläche auf, die vorzugsweise in einer Richtung parallel der Längsachse des Dampfauslasskanals verläuft. Die Richtung ist vorzugsweise in dem genannten Winkel β zu der Normalen der Ebene E geneigt. Die Wasserrückführungsfläche kann Bestandteil eines Bodenabschnitts des Auslasskanals sein. Die Wasserrückführungsfläche kann auch Bestandteil eines Wasserführungselements sein, dass entlang dieser Richtung am Bodenabschnitt des Dampfauslasskanals angeordnet ist.

Die Wasserrückführungsfläche kann insbesondere durch eine Rückführungsrinne gebildet sein, die in einer Innenwand des Dampfauslasskanals ausgebildet ist oder die durch einen Bodenabschnitt des Dampfauslasskanals gebildet ist, und die sich zwischen der Dampfaustrittsöffnung und der Heizfläche erstreckt.

Vorzugsweise weist die Wasserrückführungseinrichtung ein Wasserführungselement, insbesondere ein Stiftelement, auf, dass sich zwischen der Dampfaustrittsöffnung und der Heizfläche erstreckt. Das Wasserführungselement kann eine höhere Wärmeleitfähigkeit aufweisen als die Innenwand des Dampfauslasskanals. Das Wasserführungselement kann Metall aufweisen oder daraus bestehen. Vorzugsweise ist das Wasserführungselement in thermischem Kontakt mit der Heizfläche stehend angeordnet. Vorzugsweise ist das Wasserführungselement in der Rückführungsrinne oder im Bodenabschnitt des Dampfauslasskanals angeordnet. Durch das Wasserführungselement kann die Wasserführung bzw. Rückführung noch kontrollierter erfolgen.

Vorzugsweise ist eine Federlagerung vorgesehen, so dass das Wasserführungselement insbesondere zwischen der Dampfaustrittsöffnung und der Heizfläche federgelagert angeordnet ist und die Heizfläche kontaktiert. Dadurch wird es ermöglicht, das Wasserführungselement mit einer durch die Federung gewährten Montagetoleranz in die Wasserrückführungseinrichtung einzusetzen. Zudem wird das federgelagerte Wasserführungselement gegen die Heizfläche gedrückt, was den (thermischen) Kontakt mit der Heizfläche gewährleistet.

Vorzugsweise sind die Dampfaustrittsöffnung und die Wasserrückführungseinrichtung Bestandteile eines Verbindungselements, mit dem die Verdampfervorrichtung an der Seitenwand einer Inkubationskammer montierbar ist.

Das Verbindungselement weist vorzugsweise einen Befestigungsabschnitt auf, der insbesondere einen Plattenabschnitt, insbesondere ein Scheibenelement, ist. Der Plattenabschnitt weist insbesondere die Dampfaustrittsöffnung auf. Der Plattenabschnitt beinhaltet vorzugsweise eine Stirnwand, die den Dampfauslasskanal verschließt. Die Dampfaustrittsöffnung ist vorzugsweise azentrisch zu der Längsachse des Dampfauslasskanals in der Stirnwand angeordnet.

Das Verbindungselement weist vorzugsweise einen Eingriffsabschnitt auf, der insbesondere hohlzylinderförmig sein kann. Der Eingriffsabschnitt ist dazu eingerichtet, durch eine Öffnung in der Seitenwand einer Inkubationskammer bis in die Verdampferkammer geführt zu werden, wodurch die Verdampfervorrichtung mit der Seitenwand verbindbar ist. Im Eingriffsabschnitt verläuft vorzugsweise der Dampfauslasskanal. Der Eingriffsabschnitt erstreckt sich entlang einer Achse, die sich vorzugsweise senkrecht zu dem Plattenabschnitt erstreckt.

Die Verdampferkammer weist vorzugsweise einen Aufnahmeabschnitt auf, in dem der Eingriffsabschnitt des Verbindungselementes aufnehmbar ist. Vorzugsweise ist mindestens ein Dichtungselement vorgesehen, um diesen Eingriff abzudichten. Der Plattenabschnitt weist vorzugsweise mindestens eine Aussparung, insbesondere Bohrung, auf, so dass der Plattenabschnitt mit einem Wandabschnitt der Verdampfervorrichtung verbindbar ist, insbesondere mittels Schrauben.

Die Verdampferkammer ist ein Innenraum, der von mindestens einem Wandabschnitt begrenzt wird. Die Verdampferkammer ist vorzugsweise in einem Bauteil ausgebildet, das durch Formgebung die erforderliche Struktur aufweist. Die Formgebung erfolgt insbesondere durch ein spanendes Fertigungsverfahren oder ein Gussverfahren, insbesondere ein Kunststoff-Spritzgussverfahren. Die Größe der Verdampferkammer kann je nach Größe der Heizfläche insbesondere zwischen 1 cm³ und 50 cm³ liegen. Die Verdampferkammer ist ein geschlossener Raum, der an einem Wandabschnitt, insbesondere einem Bodenwandabschnitt, die Heizfläche aufweist. Die Verdampferkammer weist zudem die Wassereintrittsöffnung und die Dampfaustrittsöfffnung auf.

Die Verdampfervorrichtung weist vorzugsweise ein erstes Bauteil auf. Die Verdampfervorrichtung weist vorzugsweise ein zweites Bauteil auf. Das erste und das zweite Bauteil sind vorzugsweise miteinander verbunden. Das erste und das zweite Bauteil bestehen vorzugsweise aus Kunststoff. Das erste und/oder das zweite Bauteil sind vorzugsweise Spritzgussteile. Sie können aber auch durch ein spanendes Verfahren, insbesondere mittels Fräsen und Drehen, gefertigt sein. Der Kunststoff kann insbesondere Polyetheretherketon (PEEK) oder Polyphenylensulfid (PPS) sein. Das erste und das zweite Bauteil können aber auch integral gefertigt sein und stellen dann Abschnitte eines einzigen Bauteils dar.

Das erste Bauteil ist vorzugsweise in einer montierten Position mit der Seitenwand einer Inkubationskammer eines Laborgeräts verbindbar. Vorzugsweise trägt das erste Bauteil in der montierten Position die weiteren Komponenten der Verdampfervorrichtung, insbesondere das zweite Bauteil.

Vorzugsweise ist eine Isoliereinrichtung vorgesehen, die das erste und/oder zweite Bauteil umgibt. Dadurch wird die Verdampfervorrichtung und/oder die in der montierten Position angrenzende Seitenwand der Inkubationskammer des Laborgeräts thermisch isoliert. Die Isoliereinrichtung kann aus einem oder mehreren Isolierteilen bestehen, insbesondere Schaumteilen. Ein Schaumteil kann aus Silikonschaum bestehen oder Silikonschaum aufweisen.

Das erste Bauteil enthält vorzugsweise die Verdampferkammer. Die Verdampferkammer ist vorzugsweise zylinderförmig. Als Verdampferkammer wird ein Innenraum, insbesondere des ersten Bauteils, bezeichnet, der von Wandabschnitten, insbesondere gebildet durch das erste Bauteil, begrenzt wird. Diese Wandabschnitte beinhalten insbesondere eine erste Seitenwand, welche die Wassereintrittsöffnung aufweist. Die Wandabschnitte beinhalten und/oder bilden insbesondere die Dampfaustrittsöffnung und insbesondere den Dampfauslasskanal. Ein Bodenabschnitt dieser Wandabschnitte weist insbesondere eine Wandöffnung für eine Heizeinheit auf. Vorzugsweise weist ein weiterer Bodenabschnitt des Dampfauslasskanals eine parallel einer Längsachse des Dampfauslasskanals verlaufende (oder in einer Ebene verlaufend, die ebenfalls die Längsachse enthält) Aussparung auf, in der ein Wasserführungselement angeordnet ist. Diese axial verlaufende Aussparung mündet insbesondere in die Wandöffnung.

Das erste Bauteil enthält vorzugsweise mindestens einen Wandabschnitt der Verdampferkammer. Das erste und/oder das zweite Bauteil enthält vorzugsweise einen Aufnahmeraum zur Aufnahme einer Heizeinheit. Der Aufnahmeraum ist vorzugsweise zylinderförmig. Das erste Bauteil weist vorzugsweise einen Wandabschnitt der Verdampferkammer auf, der eine Wandöffnung beinhaltet. In diese Wandöffnung greift vorzugsweise die Heizeinheit ein, insbesondere die Heizfläche der Heizeinheit. Vorzugsweise bildet die Heizeinheit einen Bodenabschnitt der Verdampferkammer.

Das erste Bauteil weist vorzugsweise einen Lagerabschnitt auf, der zur Lagerung an der Seitenwand einer Inkubationskammer eingerichtet ist. Der Lagerabschnitt kann einen Zentrierabschnitt aufweisen, der formschlüssig in eine Dampfeinlassöffnung der Seitenwand der Inkubationskammer eingreift. Der Lagerabschnitt weist einen Planabschnitt auf, der insbesondere zur Anlage an der Seitenwand der Inkubationskammer eingerichtet ist.

Die Heizeinheit weist vorzugsweise einen Heizblock auf. Dieser weist vorzugsweise ein Metall auf, insbesondere Edelstahl, Aluminium oder Messing, oder besteht vorzugsweise aus einem solchen Metall. Der Heizblock weist, vorzugsweise zumindest anteilig an seiner Oberseite, die Heizfläche auf. Der Heizblock ist vorzugsweise zumindest teilweise zylinderförmig. Der Heizblock ist vorzugsweise in den, vorzugsweise zylinderförmigen, Aufnahmeraum einschiebbar. Der Heizblock weist vorzugsweise eine, insbesondere zumindest abschnittsweise zylinderförmige, Heizelementaufnahme zur Aufnahme eines Heizelements auf. Das Heizelement ist vorzugsweise eine, insbesondere zumindest abschnittsweise zylinderförmige, Heizpatrone die elektrische Anschlüsse zum Betreiben der Heizpatrone aufweist. Das Heizelement beinhaltet vorzugsweise eine elektrische Heizung, insbesondere eine Widerstandsheizung, zum Beheizen der Heizfläche. Das Heizelement und der Heizblock können auch integral gebildet sein.

Die Heizfläche weist vorzugsweise einen Zentralabschnitt auf. Die Heizfläche ist vorzugsweise zumindest abschnittsweise planar. Vorzugsweise ist der Zentralabschnitt der Heizfläche planar, insbesondere kreisförmig. Vorzugsweise bildet der Zentralabschnitt einen Bodenabschnitt der Verdampferkammer und/oder es ragt vorzugsweise der Zentralabschnitt in die Verdampferkammer hinein. Vorzugsweise weist die Heizfläche einen, insbesondere in einem Winkel χ gegenüber dem Zentralabschnitt angeordneten Seitenabschnitt auf, der sich ausgehend vom Zentralabschnitt schräg oder vertikal nach unten erstreckt, wobei insbesondere 185° < χ < 270°.

Die Heizfläche wird insbesondere durch den in die Verdampferkammer hineinragenden Kopfabschnitt eines Heizblocks gebildet. Der Kopfabschnitt hat vorzugsweise die Form eines Kegelstumpfes, dessen Oberseite insbesondere der Zentralabschnitt ist. Der Kopfabschnitt kann auch zylinderförmig sein, kugelsegmentförmig oder eine Quaderform aufweisen. Vorzugsweise verläuft die Wasserführungsfläche, insbesondere zumindest in Gravitationsrichtung gemessen, senkrecht zum Zentralabschnitt der Heizfläche.

Der Heizblock weist vorzugsweise eine Aussparung zur Aufnahme eines Temperatursensors auf. Die Heizeinheit weist vorzugsweise den Temperatursensor auf, der vorzugsweise in diese Aussparung eingesetzt ist.

Die Heizeinheit ist vorzugsweise in einem Abstand zu der Seitenwand der Inkubationskammer des Laborgeräts angeordnet, wenn die Verdampfervorrichtung an der Inkubationskammer montiert ist. Ein Planabschnitt des Verbindungselements verläuft insbesondere in einer Ebene, zu der die Heizeinheit in diesem Abstand angeordnet ist. Dieser Abstand ist die minimale Distanz zwischen Heizeinheit und Planabschnitt und wird insbesondere senkrecht zum Planabschnitt gemessen, also insbesondere bei montierter Position der Verdampfervorrichtung entlang der Horizontalen gemessen. Durch diesen Abstand wird die thermische Beeinflussung der Inkubatorkammer durch die hohe Temperatur der Heizfläche reduziert.

Die Verdampfervorrichtung kann mindestens ein Schildelement aufweisen, das bei montierter Position der Verdampfervorrichtung zwischen der Seitenwand der Inkubationskammer des Laborgeräts und der Heizeinheit angeordnet ist. Durch das Schildelement wird die Wärmeübertragung zwischen der Seitenwand des Inkubators und der Heizeinheit reduziert und so eine Isolationswirkung erzielt.

Das erste Bauteil weist vorzugsweise eine Wasseranschlussaufnahme auf, die insbesondere zylinderförmig ist. Sie mündet insbesondere in der Wassereintrittsöffnung. Die Verdampfervorrichtung weist insbesondere ein Wasseranschlusselement auf. Dieses greift insbesondere in die Wasseranschlussaufnahme ein. Vorzugsweise ist ein Dichtungselement vorgesehen, dass zwischen der Wasseranschlussaufnahme und dem Wasseranschlusselement angeordnet ist. Das Wasseranschlusselement weist insbesondere einen Eingriffsabschnitt auf, der insbesondere zylinderförmig ist und der in die Wasseranschlussaufnahme eingreift. Eine Wasserleitung, insbesondere ein Rohr- oder Schlauchelement, ist vorzugsweise zwischen dem Wasseranschlusselement und der Wasserfördereinrichtung flüssigkeitsdicht verbunden. Das Wasseranschlusselement beinhaltet insbesondere einen Wasserzuführungskanal.

Die Verdampfervorrichtung weist vorzugsweise einen Wasserzuführungskanal auf, der in die Wassereintrittsöffnung mündet, wobei insbesondere ein Öffnungsquerschnitt der Wassereintrittsöffnung kleiner ist als ein Öffnungsquerschnitt des Wasserzuführungskanals. Vorzugsweise ist zwischen Wasserzuführungskanal und Wassereintrittsöffnung eine Reservoirkammer mit einem Öffnungsquerschnitt angeordnet, der größer ist als der Öffnungsquerschnitt des Wasserzuführungskanals. Der Öffnungsquerschnitt wird jeweils senkrecht zur Flussrichtung gemessen.

Der Dampfauslasskanal erstreckt sich vorzugsweise zumindest teilweise entlang seiner Längsachse, wobei die Dampfaustrittsöffnung einen kleineren Öffnungsquerschnitt aufweist als ein senkrecht zur Längsachse gemessener Querschnitt des Dampfauslasskanals. Der Dampfauslasskanal kann zylinderförmig oder konusförmig sein, oder kann die Form eines Kegelschnitts oder eines schiefen Kegels aufweisen.

Der Öffnungsquerschnitt der Dampfaustrittsöffnung weist vorzugsweise einen maximalen Durchmesser auf, der zwischen 0,5 mm und 20 mm beträgt, vorzugsweise zwischen 2 mm und 10 mm, vorzugsweise zwischen 3 mm und 5 mm. Die Dampfaustrittsöffnung kann auch mehrere kleinere Öffnungen mit einem maximalen Öffnungsquerschnitt kleiner als 2 mm aufweisen oder durch ein Netzelement gebildet oder von diesem bedeckt sein. Ein Netzelement reduziert die unerwünschte Tropfenbildung an der Dampfaustrittsöffnung.

Vorzugsweise ist die Dampfaustrittsöffnung azentrisch zu der Längsachse des Dampfauslasskanals angeordnet. Dabei ist vorzugsweise die Dampfaustrittsöffnung oberhalb der einen Boden der Verdampferkammer bildenden Heizfläche und insbesondere oberhalb der Längsachse angeordnet ist, wenn die Verdampfervorrichtung im bestimmungsgemäßen Gebrauch an einer vertikalen Seitenwand einer Inkubationskammer montiert ist. Diese Anordnung hat sich besonders bewährt, um eine Tropfenbildung zu vermeiden. Je mehr Kondensattropfen gebildet werden, desto schwieriger wird eine präzise Kontrolle der Dampfabgabe der Verdampfervorrichtung.

Die Wasserfördereinrichtung wird vorzugsweise durch eine Pumpeneinrichtung gebildet.

Die Pumpeneinrichtung kann eine Mikrodosierpumpe sein, eine Membranpumpe, eine Peristaltikpumpe, eine Schneckenpumpe oder eine Piezo-Pumpe. Die Wasserfördereinrichtung kann auch für eine Wasserförderung durch Schwerkraft eingerichtet sein. Dazu weist die Wasserfördereinrichtung vorzugsweise eine Drosseleinrichtung und einen Wasserzufluss auf. Die Drosseleinrichtung ist vorzugweise eingerichtet, Wasser von dem Wasserzufluss zu erhalten und nach einer Drosselung des Wasserdrucks oder Wasserstroms das Wasser auszugeben. Die Drosseleinrichtung liegt vorzugsweise unterhalb des Wasserzuflusses und erlaubt einen durch Drosselung dosierten Wasserfluss entlang der Gravitationsrichtung. Das zufließende Wasser kann insbesondere von einem oberhalb der Drosseleinrichtung bzw. der Wasserfördereinrichtunganordenbaren Wasserreservoir oder Wasseranschluss stammen. Die Drosseleinrichtung ist insbesondere elektrisch steuerbar und dazu eingerichtet, die Größe eines Wasserleitungsquerschnitts und damit den Fluss des Wassers zu steuern.

Vorzugsweise ist die Pumpeneinrichtung dazu eingerichtet, mit einer definierten Pumpleistung betrieben zu werden. Dazu kann insbesondere ein Pumpleistungsparameter für die Pumpeneinrichtung vorgebbar sein. Dieser kann insbesondere eine Pumpfrequenz sein, ein Tastverhältnis im Falle einer PWM-Ansteuerung der Pumpe, eine Spannung, eine Stromstärke, insbesondere der Zeitverlauf eines solchen Signals. Vorzugsweise ist die Pumpeneinrichtung zur Erzeugung einer Anzahl von Pumpenhüben eingerichtet, insbesondere derart, dass diese Anzahl von Pumpenhüben in einer verdampfbaren Wassermenge resultiert, der durch die Wassereintrittsöffnung auf die Wasserführungseinrichtung befördert wird und zur Heizfläche gelangt. Die Pumpleistung, insbesondere eine Anzahl von Pumpenhüben, kann vordefiniert sein oder kann geregelt sein, und kann insbesondere in Abhängigkeit von einer gemessenen Heizleistung der Heizfläche regelbar sein.

Es ist auch möglich, dass die Pumpeneinrichtung zur Erzeugung eines Pumpenhubs eingerichtet, insbesondere derart, dass -im Wesentlichen- jeder Pumpenhub in einer verdampfbaren Wassermenge bzw. einem verdampfbaren Tropfen resultiert, der durch die Wassereintrittsöffnung auf die Wasserführungseinrichtung befördert wird und zur Heizfläche gelangt. Damit ist eine kontrollierbare Wasserverdampfung gewährleistet, die in inkrementellen Dosiervolumina erfolgt, entsprechend einem Tropfen oder einer als Wasserfilm abgegeben Wassermenge, die zur Heizfläche gelangt.

Vorzugsweise ist die Wasserfördereinrichtung durch mindestens eine der folgenden technischen Daten gekennzeichnet:
a) das Wasserfördervolumen beträgt zwischen 1 µl und 50 µl, vorzugsweise zwischen 5 µl und 20 µl, vorzugsweise zwischen 10 µl und 20 µl, vorzugsweise zwischen 7 µl und 15 µl, vorzugsweise zwischen 9 µl und 11 µl;
b) die minimale Wasserförderrate beträgt zwischen 0.5 µl/h und 100 µl/h, vorzugsweise zwischen 0.5 µl/h und 20 µl/h, vorzugsweise zwischen 0.5 µl/h und 10 µl/h.

Vorzugsweise die Verdampfervorrichtung eine elektronische Steuereinrichtung auf. Diese ist insbesondere dazu eingerichtet, die Wasserfördereinrichtung und die Heizfläche zu steuern, Diese Steuereinrichtung ist vorzugsweise dazu eingerichtet, die Dampfabgaberate der Verdampfervorrichtung zu steuern. Diese Steuerung erfolgt mit dem Ziel der Regelung der der Luftfeuchte in einer mit der Dampfaustrittsöfffnung verbindbaren Inkubationskammer, insbesondere indem die Wasserförderleistung der Wasserfördereinrichtung frequenzgesteuert durch ein elektrisches Signal mit einer Eingangsfrequenz definiert wird.

Die Wasserfördereinrichtung kann auch als Dosiereinrichtung ausgebildet sein, die dazu eingerichtet ist, ein vorgegebenes Dosiervolumen auszugeben, das zur Heizfläche gelangt und dort verdampft wird. Dies kann insbesondere realisiert sein, indem die Dosiereinrichtung mindestens einen Kolben aufweist, der das Wasservolumen definiert aus der Kolbenkammer verdrängt und ausgibt, insbesondere mittels Wasserführungseinrichtung, insbesondere Rohrstück oder Kapillare, direkt zur Heizfläche ausgibt.

Die Steuereinrichtung ist insbesondere mit einem Temperatursensor verbunden, der angeordnet ist, um die Temperatur der Heizfläche zu erfassen.

Die Steuereinrichtung ist vorzugsweise dazu eingerichtet, die Temperatur der Heizfläche auf eine Zieltemperatur einzustellen bzw. zu regeln, die insbesondere aus dem Bereich zwischen 100 °C bis 250 °C ausgewählt ist und vorzugsweise zwischen 140 °C und 200°C, vorzugsweise zwischen 170 °C und 190°C beträgt.

Die elektrische Steuereinrichtung weist insbesondere eine Datenverarbeitungseinrichtung auf und ist vorzugsweise dazu programmiert, eine Temperatur einer Heizeinheit, insbesondere der Heizfläche, zu erfassen und insbesondere in Abhängigkeit von dieser Temperatur die Leistung der Heizeinheit einzustellen. Vorzugsweise ist die Steuereinrichtung dazu programmiert, eine Temperatur der Heizeinheit, insbesondere eines Heizblocks oder insbesondere der Heizfläche auf eine gewünschte, insbesondere konstante, Zieltemperatur zu regeln. Vorzugsweise ist die Steuereinrichtung dazu programmiert, einen Heizregelkreis zu bilden, der dazu eingerichtet ist, die mittels eines Temperatursensors gemessene Temperatur eines Heizelements des Verdampfers auf eine konstante Zieltemperatur zu regeln, bei der ein mit dem Heizelement in Kontakt kommendes Wasservolumen verdampft wird und dabei dem Heizelement Wärme entzieht,

Die elektronische Steuereinrichtung ist vorzugsweise dazu programmiert, mindestens eine Funktion der Verdampfervorrichtung zu steuern, insbesondere die Zuführung von Wasser durch die Wassereintrittsöffnung mittels der Wasserfördereinrichtung, und/oder das Heizen der Heizfläche, und/oder das Messen einer Temperatur des Heizelementes. Die elektronische Steuereinrichtung ist vorzugsweise dazu programmiert, Daten zu erfassen, die Informationen eines Sensors des Laborgeräts beinhalten, und insbesondere in Abhängigkeit von diesen Daten die mindestens eine Funktion der Verdampfervorrichtung zu steuern. Der Sensor kann insbesondere ein Luftfeuchtesensor sein, der die Luftfeuchte in der Inkubationskammer des Laborgeräts misst. Der Sensor kann auch ein Türsensor sein, der erfasst, ob eine die Inkubationskammer verschließende Türe geöffnet oder geschlossen ist. Insbesondere kann vorgesehen sein, die Dampfabgaberate der Verdampfervorrichtung in Abhängigkeit von diesen Daten zu ändern, beispielsweise um nach einer Öffnung der Türe (bei dann wieder geschlossener Türe) die Dampfabgaberate schnell zu erhöhen, um die gewünschte relative Luftfeuchte, z.B. 95%, in der Inkubationskammer möglichst schnell wiederherzustellen.

Die Funktionen der Steuereinrichtung sind insbesondere durch Programmcode und/oder durch elektronische Schaltkreise implementiert. Die Steuereinrichtung kann einen Mikrocontroller, eine Recheneinheit (CPU) zum Verarbeiten von Daten bzw. einen Mikroprozessor aufweisen, die jeweils der Datenverarbeitungseinrichtung zugeordnet sein können.

Die Steuereinrichtung kann als selbständig arbeitendes Bauteil ausgebildet sein, dass die Funktionen der Verdampfervorrichtung steuert, das aber insbesondere nicht eine oder mehrere Funktionen des Laborgeräts steuert, mit dem die Verdampfervorrichtung verbunden ist oder dessen Bestandteil die Verdampfervorrichtung vorzugsweise ist.

Die Steuereinrichtung kann aber auch durch eine solche Steuereinrichtung gebildet sein, die außer den Funktionen der Verdampfervorrichtung auch mindestens eine, mehrere oder alle Funktionen des Laborgeräts steuert, mit dem die Verdampfervorrichtung verbunden ist oder dessen Bestandteil die Verdampfervorrichtung vorzugsweise ist. Eine der Funktionen des Laborgeräts ist insbesondere die Regelung der Temperatur in der Inkubationskammer des Laborgeräts, oder die Regelung der Gaszusammensetzung in der Inkubationskammer, insbesondere der CO₂-Konzentration. Eine der Funktionen des Laborgeräts ist insbesondere auch die Steuerung eines Benutzerschnittstellenmoduls des Laborgeräts, das dem Benutzer Informationen anzeigt, insbesondere über Sensorwerte zu in/an der Inkubationskammer gemessenen physikalischen oder chemischen Größen.

Die Erfindung betrifft ferner ein Laborgerät zur Inkubation von Proben in einer Inkubationskammer, insbesondere einen Inkubator zur Inkubation lebender Zellkulturen oder einen Shaker mit Inkubationsfunktion, welcher eine erfindungsgemäße Verdampfervorrichtung aufweist.

Der Inkubator ist ein Laborgerät bzw. ein Laborinkubator. Ein Inkubator bezeichnet insbesondere ein Laborgerät mit einer Inkubatorkammer (Inkubationskammer), deren Atmosphäre vom Inkubator auf eine vorgegebene Zieltemperatur regelbar ist bzw. geregelt wird. Insbesondere handelt es sich um ein Laborgerät, mit dem kontrollierte Klimabedingungen (Temperatur, Gas und Luftfeuchte) für verschiedene biologische Entwicklungs- und Wachstumsprozesse geschaffen und erhalten werden können. Der Inkubator kann ein Schüttelinkubator (Shaker), also ein Inkubator mit einer Bewegungseinrichtung zur Bewegung von in der Inkubatorkammer angeordneten Objekten, sein oder diesen beinhalten, und kann ein Microbial-Inkubator (auch ohne CO₂) sein. Der Inkubator kann insbesondere als eine Zellkultivierungsvorrichtung ausgebildet sein. Der Inkubator dient insbesondere der Schaffung und Erhaltung eines Mikroklimas mit geregelten Gas-, und/oder Luftfeuchtigkeits- und/oder Temperatur-Bedingungen in der Inkubatorkammer, wobei diese Behandlung zeitabhängig sein kann. Der Labor-Inkubator, insbesondere eine Behandlungseinrichtung des Labor-Inkubators, kann insbesondere einen Zeitgeber aufweisen, insbesondere eine Zeitschaltuhr, eine Heiz-/Kühleinrichtung und vorzugsweise eine Einstellung für die Regelung eines der Inkubatorkammer zugeführten Austauschgases, eine Einstelleinrichtung für die Zusammensetzung des Gases in der Inkubatorkammer des Inkubators, insbesondere zur Einstellung des CO₂ und/oder des O₂ und/oder des N₂-Gehalts des Gases und/oder eine Einstelleinrichtung zur Einstellung der Luftfeuchtigkeit in der Inkubatorkammer des Inkubators.

Der Inkubator, insbesondere eine Behandlungseinrichtung des Inkubators, weist insbesondere die Inkubatorkammer auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur im Inkubator geregelt werden. Je nach Ausführungsform kann darüber auch die Luftfeuchte geregelt werden.

Vorzugsweise weist der Inkubator die erfindungsgemäße Verdampfervorrichtung auf, mittels der die Luftfeuchtigkeit in der Atmosphäre der Inkubatorkammer eingestellt wird.

CO₂-Inkubatoren dienen insbesondere der Kultivierung tierischer bzw. humaner Zellen.

Inkubatoren können Wendevorrichtungen zum Wenden des mindestens einen Zellkulturbehälters und/oder eine Schütteleinrichtung zum Schütteln bzw. Bewegen des mindestens einen Zellkulturbehälters aufweisen. Der erfindungsgemäße Inkubator ist insbesondere nicht ein Bioreaktor oder Fermenter.

Der Inkubator kann mindestens eine Sensoreinrichtung aufweisen. Eine Sensoreinrichtung weist insbesondere mindestens einen Temperatursensor auf, vorzugsweise eine Vielzahl von Temperatursensoren. Ein Temperatursensor kann beispielsweise ein Pt 100- oder Pt 1000-Temperaturfühler sein. Eine Sensoreinrichtung weist vorzugsweise einen Sensor zur Ermittlung einer relativen Gaskonzentration auf, insbesondere zur Ermittlung des Gehalts an CO₂ und/oder O₂ und/oder N₂. Eine Sensoreinrichtung weist vorzugsweise einen Sensor zur Ermittlung der relativen Luftfeuchtigkeit auf.

Ein Inkubator weist vorzugsweise eine bzw. eine einzige Inkubatorkammer auf. Diese kann in Kompartimente unterteilt sein. Kompartimente können durch -insbesondere gelochte-Lagerplatten getrennt sein, wobei insbesondere ein Gasaustausch zwischen den Kompartimenten ermöglicht ist.

Die Inkubatorkammer weist Kammerwände bzw. Kammerinnenwände und genau eine oder mindestens eine Kammeröffnung auf, über die die Objekte bzw. Zellkulturbehälter im Inneren der Inkubatorkammer platzierbar und entnehmbar sind. Diese Kammeröffnung ist durch ein mit der Inkubatorkammer und/oder dessen Gehäuseeinrichtung beweglich verbundenes Verschlusselement verschließbar, insbesondere eine mittels Scharnier oder Schwenkeinrichtung an der Inkubatorkammer oder der Gehäuseeinrichtung beweglich montierte Inkubatortüre, insbesondere eine oder mehrere Kammertüren. Ein Inkubator kann eine oder mehrere Innentüren aufweisen, die insbesondere transparent sein können, und kann eine -insbesondere nicht transparente- Außentür aufweisen, welche insbesondere die Inkubatorkammer und gegebenenfalls mindestens einer innere Inkubatortüre, welche die Kammeröffnung verschließt bzw. öffnet, zur Umgebung hin thermisch isoliert. Eine Gehäuseeinrichtung kann Rahmenteile und/oder Rahmenwände und/oder Gehäusewände beinhalten. Eine Schwenkeinrichtung kann Schwenkarme beinhalten, mit denen die Inkubatortüre von der Kammeröffnung wegschwenkbar ist, insbesondere vollständig wegschwenkbar ist.

In der verschlossenen Position der Kammeröffnung ist das Innere der Inkubatorkammer gegen die Umgebung vorzugsweise in der Weise isoliert, so dass im Inneren eine gewünschte, vom Inkubator gesteuerte Temperatur bzw. Atmosphäre einstellbar, insbesondere regelbar ist. In der geöffneten Position der Kammeröffnung ist über diese Öffnung ein Gasaustausch zwischen der Umgebung des Inkubators und dem Inneren der Inkubatorkammer möglich. Die Kammeröffnung befindet sich typischer Weise in einer die Kammeröffnung umlaufenden Frontwand des Inkubators.

Die Inkubatorkammer weist vorzugsweise mehrere Wände bzw. Innenwandflächen auf, die insbesondere einstückig und insbesondere kantenfrei miteinander verbunden sein können. Die Wände bzw. Innenwandflächen sind vorzugsweise im Wesentlichen planar geformt, können aber auch alle oder zum Teil eine geschwungene Form aufweisen. Die Inkubatorkammer ist vorzugsweise quaderartig geformt, kann aber auch anders geformt sein, z.B. sphärisch, ellipsoid, polyederförmig. Die Wände bzw. Innenwandflächen sind vorzugsweise aus einem korrosionsarmen Material gefertigt, insbesondere Edelstahl, , oder ein Kunststoff, insbesondere ein Verbundkunststoff. Dadurch wird die Reinigung/ Desinfektion des Kammerinneren erleichtert. Unabhängig von der Kammeröffnung, die dem Bestücken / Entnehmen von Objekten bzw. Zellkulturbehältern dient, kann die Inkubatorkammer mindestens einen Port zum Durchführen einer entsprechend dimensionierten Vorrichtung bzw. einer Kabelverbindung aus dem Inneren der Inkubatorkammer an deren Außenseite oder in die Umgebung des Inkubators aufweisen.

Eine Seitenwand der Inkubatorkammer weist insbesondere eine Dampfeintrittsöffnung auf, die mit der Dampfaustrittsöffnung der Verdampfervorrichtung verbunden ist, insbesondere fluiddicht. Diese Seitenwand der Inkubatorkammer weist insbesondere auch einen Befestigungsabschnitt zur Befestigung der Verdampfervorrichtung auf. Der Befestigungsabschnitt kann die Dampfeintrittsöffnung beinhalten, in der vorzugsweise ein Verbindungselement zur Verbindung der Verdampfervorrichtung mit der Seitenwand angeordnet ist.

Eine typische Größe des Inneren einer Inkubatorkammer liegt zwischen 50 und 400 Litern (dm³).

Der Inkubator kann genau eine Inkubatorkammer aufweisen, kann aber auch mehrere Inkubatorkammern aufweisen, deren Luftfeuchte der Atmosphäre (oder: Temperatur, relative Gaskonzentration) insbesondere individuell oder gesammelt einstellbar sein kann. Ein Inkubator kann mehrere Inkubatorkammern aufweisen, die jeweils eine eigene Kammeröffnung und eine eigene Kammertüre zum Verschließen der Kammeröffnung aufweisen können. Jede dieser Inkubatorkammern, oder eine Gruppe dieser Inkubatorkammern, kann mit einer erfindungsgemäßen Verdampfervorrichtung verbunden sein, um die Luftfeuchte individuell oder gruppenweise einzustellen bzw. zu regeln.

Der Inkubator kann eine Gehäuseeinrichtung, insbesondere ein Gehäuse, aufweisen, die/das die Inkubatorkammer teilweise oder vollständig umgibt. Eine erfindungsgemäße Verdampfervorrichtung ist vorzugsweise, insbesondere neben der Inkubatorkammer, innerhalb des Gehäuses angeordnet. Das Gehäuse kann im Wesentlichen quaderförmig ausgebildet sein, und kann insbesondere derart ausgebildet sein, dass der Inkubator stapelbar ist.

Die Erfindung betrifft auch ein System aus einer Anzahl N>1 an Inkubatoren, und mindestens einer erfindungsgemäßen Verdampfervorrichtung, insbesondere einer Anzahl M>=1 von erfindungsgemäßen Verdampfervorrichtungen, vorzugsweise M=N, die jeweils individuell mit einer Inkubatorkammer verbunden sind. Vorzugsweise weist das System ein Wasserreservoir auf, das insbesondere oberhalb eines Stapels der N Inkubatoren angeordnet ist. Bei dem Einsatz einer Pumpeneinrichtung als Wasserfördereinrichtung kann das Wasserreservoir auch unterhalb der Pumpeneinrichtung angeordnet sein und erhöht somit die Flexibilität bei der Aufstellung. Das Wasserreservoir ist mit der Wasserfördereinrichtung jeder der Verdampfervorrichtungen verbunden. Das System ist insbesondere so eingerichtet, dass im Betrieb mehrerer dieser Inkubatoren Wasser aus diesem einen Wasserreservoir von den Verdampfervorrichtungen dieser Inkubatoren verdampft wird.

Die Erfindung betrifft auch ein Verfahren zum Erzeugen eines Wasserdampfes zur Befeuchtung der Atmosphäre einer Inkubationskammer eines Laborgeräts, insbesondere eines Inkubators zur Inkubation lebender Zellkulturen, mittels einer eine Heizfläche aufweisenden Verdampfervorrichtung, insbesondere einer erfindungsgemäßen Verdampfervorrichtung, wobei Wasser mittels einer Wasserführungseinrichtung an der Wasserführungseinrichtung entlang fließend bis zu der Heizfläche geführt wird, die das Wasser verdampft.

Weitere bevorzugte Ausgestaltungen der erfindungsgemäßen Gegenstände, insbesondere des erfindungsgemäßen Verfahrens, lassen sich aus der Beschreibung der erfindungsgemäßen Verdampfervorrichtung bzw. des Laborgeräts mit Inkubationsfunktion und deren bevorzugten Ausgestaltungen entnehmen. Ferner ergeben sich weitere Ausgestaltungsoptionen der Erfindung aus den Ausführungsbeispielen in den Figuren. Gleiche Teile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:
Fig. 1a zeigt einen Querschnitt durch eine perspektivische Seitenansicht der Verdampfervorrichtung gemäß einem Ausführungsbeispiel der Erfindung, in montierter Position an der Seitenwand einer Inkubationskammer eines Inkubators, ohne die Wasserfördereinrichtung.
Fig. 1b zeigt einen Querschnitt durch eine perspektivische Seitenansicht der Verdampfervorrichtung gemäß Fig. 1a mit modifiziertem Dampfauslasskanal, in montierter Position an der Seitenwand einer Inkubationskammer eines Inkubators, ohne die Wasserfördereinrichtung.
Fig. 2a zeigt einen Querschnitt durch eine perspektivische Seitenansicht einer Verdampfervorrichtung gemäß einem Ausführungsbeispiel der Erfindung.
Fig. 2b zeigt ein Detail der Fig. 2a.
Fig. 3a zeigt eine perspektivische Seitenansicht der Verdampfervorrichtung der Fig. 2, aus einem Ansichtswinkel schräg von vorne und unten, ohne die Wasserfördereinrichtung.
Fig. 3b zeigt eine perspektivische Seitenansicht der Verdampfervorrichtung der Fig. 3a, aus einem Ansichtswinkel schräg von hinten und oben, ohne die Wasserfördereinrichtung.
Fig. 4a bis 4 e zeigen jeweils ein Ausführungsbeispiel des den Dampfauslasskanal aufweisenden Verbindungselements, das zur Verbindung der Verdampfervorrichtung gemäß Fig. 1a bis 3b mit der Seitenwand einer Inkubationskammer verwendbar ist.
Fig. 5a zeigt in schematischer Frontansicht ein Ausführungsbeispiel der Verdampfervorrichtung gemäß einem Ausführungsbeispiel der Erfindung.
Fig. 5b zeigt in schematischer Frontansicht ein System aus einem Stapel von Verdampfervorrichtungen gemäß Fig. 5a, die mit einem gemeinsamen Wasserreservoir verbunden sind.
Fig. 5c zeigt ein Ausführungsbeispiel eines erfindungsgemäßen Laborgeräts, das ein Inkubator ist, an dessen Inkubatorkammerwand die Verdampfervorrichtung der Fig. 2 montiert ist.

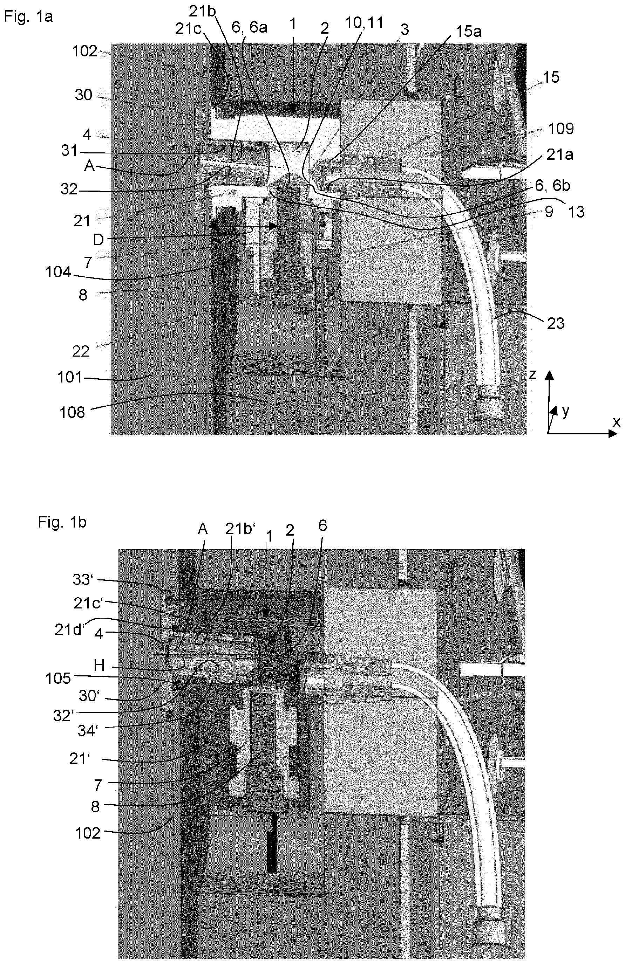

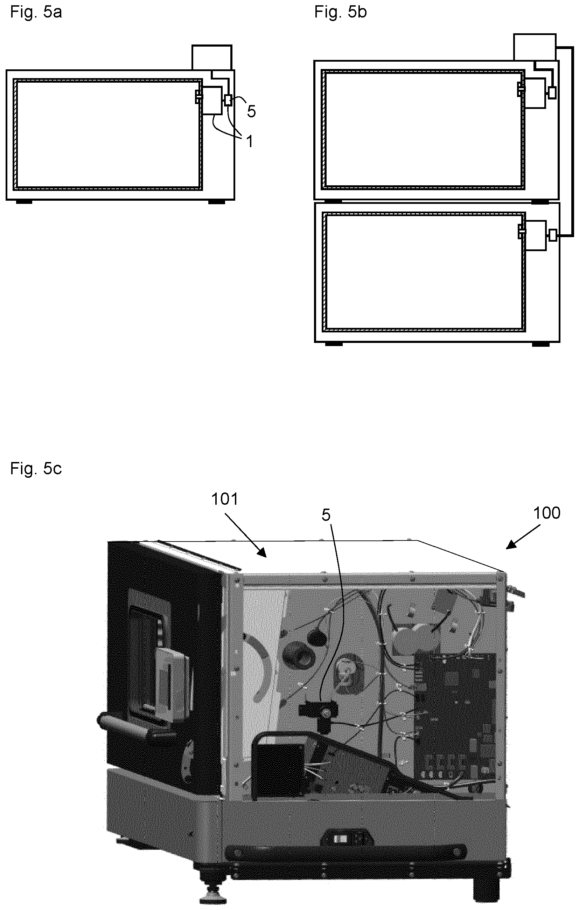

Fig. 1a zeigt die Verdampfervorrichtung 1, in montierter Position an der Seitenwand 102 einer Inkubationskammer 101 eines COz-Inkubators 100 zur Inkubation von Zellkulturen, der in Fig. 5c gezeigt ist. Die negative z-Richtung entspricht der Gravitationsrichtung. Die Verdampfervorrichtung 1 dient der Befeuchtung der Inkubatoratmosphäre in der Inkubationskammer. Die Verdampfervorrichtung 1 weist eine Verdampferkammer 2 auf, deren Bodenabschnitt eine Heizfläche 6 enthält, die mit dem Innenraum der Verdampferkammer in Kontakt ist und die somit in der Verdampferkammer 2 angeordnet ist und mit der ein mit der Heizfläche in Kontakt kommendes Wasser verdampfbar ist. Die Verdampfervorrichtung 1 weist ein erstes Bauteil 21 auf. Dieses enthält die hier durch Drehen aus einem gefrästen Bauelement gefertigte Verdampferkammer 2, welche eine Wassereintrittsöffnung 3 zur Zuführung von flüssigem Wasser in die Verdampferkammer und eine mit dem Innenraum einer Inkubatorkammer des Inkubators verbindbare Dampfaustrittsöffnung 4 aufweist.

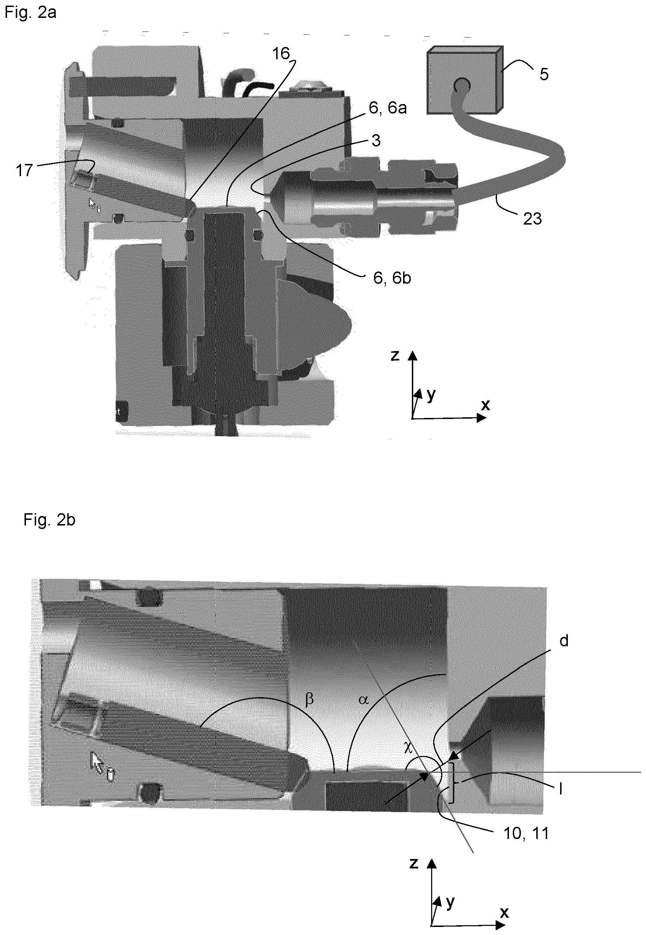

Die Wasserfördereinrichtung 5, eine Mikromembranpumpe, mit der Wasser zu der Wassereintrittsöffnung beförderbar ist, ist in Fig. 2 gezeigt (nicht aber in Fig. 1a). Die Wasserfördereinrichtung 5 wird über die Wasserleitung 23 an das Wasseranschlusselement 15 angeschlossen. Das Wasseranschlusselement 15 weist eine zylinderförmigen Mündungsabschnitt 15a auf, der in einem zylinderförmigen Aufnahmeraum 21a des ersten Bauteils 21 aufgenommen ist, der in die Wassereintrittsöffnung 3 mündet; sowohl der Mündungsabschnitt 15a als auch der Aufnahmeraum 21a münden somit in der Wassereintrittsöffnung 3.

Die Verdampfervorrichtung 1 weist eine Wasserführungseinrichtung 10 mit einer Wasserführungsfläche 11 auf, die durch einen Innenwandabschnitt 11 der Verdampferkammer gebildet wird. Mittels der Wasserführungseinrichtung 10 wird das durch die Wassereintrittsöffnung 3 in die Verdampferkammer 2 eingetretene Wasser bis zu der Heizfläche 6 an der Wasserführungseinrichtung 10 entlang fließend geführt.

Im unteren, zweiten Bauteil 22, das mit dem ersten Bauteil 21 verbunden ist, befindet sich die Heizpatrone 8, die in dem Heizblock 7 eingehaust ist. Zur Temperaturregelung ist ein Temperatursensor 9 an dem Heizblock 7 befestigt. Das Wasser gelangt über die kleine Drosselbohrung 3 in die Verdampferkammer 2. Anschließend läuft der eingeleitete Tropfen an der Wandung 11 herunter auf den beheizten Heizblock und wird dort verdampft.

Die Wasserfördereinrichtung, die Pumpe, ist hier so gewählt und eingerichtet, dass eine fein geregelte Pumpleistung eine geringe verdampfbare Wassermenge zur Heizfläche führt, was eine entsprechende Dampfmenge erzeugt, , um den relativen Luftfeuchtigkeitswert (auch: rH-Wert) möglichst fein und gleichmäßig einregeln zu können. Würden sich erst große Tropfen bilden, bevor es zu einem Ablösen und Verdampfen kommt, kann es durch das explosionsartige Verdampfen dieses Tropfens zum Herausschleudern von kleineren Tropfen führen. Zudem ist die Gefahr größer, dass es zu einem Überschießen des rH-Werts in der Kammer kommt. In den Vorversuchen der Erfinder hatte sich gezeigt, dass es vorteilhaft ist, wenn der Tropfen ohne Fall zur Heizfläche gelangt, indem er insbesondere unmittelbar an der Wand 11 herunterläuft. Die Oberflächenspannung des Wassers wird in dieser Anordnung früher überwunden, so dass es zu einem Ablaufen des Wassers in Richtung des Heizblocks 7 kommt.

In Fig. 1a auf der linken Seite befindet sich die Dampfaustrittsöffnung 4 in die Inkubatorkammer 101. Die Dampfaustrittsöffnung 4 befindet sich oberhalb der Längsachse A des Dampfauslasskanals 31. Kondensierendes Wasser in der Dampfaustrittsöffnung 4 soll über die schräge Bodenwand 32 zurück zum Heizblock gefördert werden. Zwischen Inkubatorkammer 101 und Heizpatrone 8 ist ein Abstand D vorgesehen, um einen Übergriff der Temperatur auf die Kammerwand 102 zu reduzieren. Zum weiteren Wärmeschutz kann in den Hohlraum 104 Isoliermaterial eingelegt werden, zudem können dort parallel zur Seitenwand ausgerichtete Plattenelemente 25 des Bauteils 21/21' zur thermischen Abschirmung zwischen dem Heizblock 7 und der Seitenwand 102 vorgesehen werden. Zudem sind beide Bauteile (Gehäuseteile) 21, 22 aus einem schlecht wärmeleitenden Material (hier Kunststoff PEEK) gefertigt. Um die Energieeffizienz zu erhöhen sowie zur besseren Entkopplung von Umgebungseinflüssen ist der Verdampfer nach außen hin durch Silikonisolierschaumteile 108, 109 isoliert. Die Isolierung 108, 109 kann zu Reparaturzwecken einfach entnommen werden.

Fig. 1b zeigt die Verdampfervorrichtung 1 mit modifiziertem Dampfauslasskanal 4' und modifizierter Einhausung 21', in montierter Position an der Seitenwand einer Inkubationskammer eines Inkubators, ohne die Wasserfördereinrichtung. Das Verbindungselement 30' enthält, analog dem Verbindungselement 30, den Dampfauslasskanal 31'. Dessen gegenüber der Horizontalen H schräger Bodenabschnitt 32' führt von der Dampfaustrittsöffnung 4 zur Heizfläche 6. Diese bildet dort den Bodenabschnitt der Verdampferkammer 2.

Das Bauteil 21' weist einen zylinderförmigen Aufnahmeraum 21b' auf, in den der im Wesentlichen zylinderförmige Eingriffsabschnitt 34` des Verbindungselements 30` formschlüssig eingreift. Die seitliche Öffnung des Aufnahmeraums 21b' wird durch einen optionalen ringförmigen Vorsprung 21d` gebildet, der in die Öffnung 105 der Seitenwand 102 der Inkubatorkammer 101 eingreift und so in der z-y-Ebene fixiert wird. Das Verbindungselement 30' weist den im Wesentlichen scheibenförmigen Kopfabschnitt 33' auf, der sich parallel der Seitenwand 102 und senkrecht zur Längsachse H des zylinderförmigen Eingriffsabschnitt 34` erstreckt. Zur Befestigung der Verdampfervorrichtung 1 an der Seitenwand wird das Bauteil 21' (bzw. die miteinander verbundenen Bauteile 21 und 22 in Fig. 1a) in die Öffnung 105 gesteckt und von der Innenseite der Inkubatorkammer wird das Verbindungselement 30' in den Aufnahmeraum 21b' gesteckt. Zur endgültigen Fixierung der Verdampfervorrichtung entlang der (positiven) x-Achse (in horizontaler Richtung) an der Seitenwand 102 wird der Kopfabschnitt 33' mithilfe von Bohrungen im Kopfabschnitt 33', der Seitenwand 102 und dem Bauteil 21' mit dem Bauteil 21' verschraubt.

Die Abdichtung der Verdampferkammer 2 erfolgt hier mittels zweier O-Ringe zwischen dem Eingriffsabschnitt 34` und dem Aufnahmeraum 21b'. Ähnlich erfolgt die Abdichtung des Wasseranschlusselements 15 gegenüber dem Aufnahmeraum 21a des ersten Bauteils 21 (21') durch einen O-Ring. Auch der Eingriff des Heizblocks 7 in die Öffnung 13 im Bodenwandabschnitt der Verdampferkammer wird durch einen O-Ring abgedichtet. Auf diese Weise ist die Verdampferkammer 2 prinzipiell nur zu der Dampfaustrittsöffnung 4 geöffnet.

Fig. 2a zeigt die Verdampfervorrichtung 1 mit einem Verbindungselement 30" gemäß einem weiteren Ausführungsbeispiel der Erfindung. In Verbindung mit dem Detailausschnitt in Fig. 2b lassen sich die Maßnahmen gut nachvollziehen, die zur Verbesserung der kontrollierten Dampfabgabe der Verdampfervorrichtung 1 beitragen.

Eine besonders kleine Wassereintrittsöffnung 3 (Durchmesser 1,5 mm) erlaubt eine feine Dosierung des Wassereintritts.

Die vertikal, also in Richtung der Gravitation, bzw. senkrecht gegenüber dem planaren Zentralabschnitt 6a der Heizfläche 6 angeordnete Wasserführungsfläche 11 sorgt dafür, dass kleinste Wasservolumina von z.B. 10-20 Mikroliter (vorzugsweise entsprechend einem Pumpenhub) kontrolliert an der Wasserführungsfläche 11 entlanglaufen, bis sie mit dem Rand 6b der Heizfläche in Kontakt treten und bei 160°C - 180°C Temperatur der Heizfläche verdampft werden. Ein Leidenfrost-Effekt kann auf diese Weise verhindert werden. Die Wasserführungsfläche ist vorzugsweise so angeordnet, dass ein aus der Wassereintrittsöffnung 3 in die Verdampferkammer 2 eingetretenes Wasservolumen gravitationsgetrieben an der Wasserführungsfläche 11 entlangläuft. Dazu ist ein Winkel α zwischen Wasserführungsfläche 11 und der Horizontalen erforderlich, der den Wasserfluss bewirkt. Bevorzugt ist 80° < α < 180°, insbesondere 85° < α < 175°. Ein "Überhängen" der Wasserführungsfläche 11 bei Winkeln z.B. 80° < α < 90° ist möglich, solange das Wasservolumen hinreichend an der Oberfläche haftet, was bei eher hydrophilen Oberflächen der Fall ist.

Die Wassereintrittsöffnung 3 liegt nahe an der Heizfläche: die minimale Distanz d beträgt hier 2 mm und beträgt vorzugsweise zwischen 20 mm und 0 mm. Die Fließstrecke I für einen aus der Wassereintrittsöffnung 3 in die Verdampferkammer 2 eingetretenes Wasservolumen beträgt hier l=3 mm. Durch die Nähe der Heizfläche und durch die Erwärmung der Wasserführungsfläche 11 wird der Wassertropfen auch bereits bei Annäherung an die Heizfläche erwärmt, was vorteilhaft für ein kontinuierliches Verdampfen des Wasservolumens ist.

Die Kegelstumpfform des Kopfteils des Heizblocks 7, das hier in die Verdampferkammer 2 hineinragt, bietet mit dem gegenüber dem planaren Zentralabschnitt 6a geneigten Seitenabschnitt 6b einen weiteren Vorteil. Ein an der Wasserführungsfläche 11 herabfließendes Wasservolumen kommt aufgrund der Neigung der Heizfläche 6b dieser Heizfläche immer näher. Einerseits wird das Wasservolumen durch Adhäsion an der Wasserführungsfläche 11 gehalten, andererseits der Heizfläche 6b weiter angenähert. Ein herabgleitender Tropfen wird so im konisch zulaufenden Spalt zwischen Heizfläche 6b und Wasserführungsfläche 11 durch Kontakt mit der Heizfläche effektiv verdampft und verringert damit seinen Durchmesser, verliert aber seine Haftung an der Wasserführungsfläche 11 nicht. Es kann deshalb eine wirksame Maßnahme sein, die Wasserführungsfläche gegenüber der Heizfläche so in einem spitzen Winkel δ anzuordnen, dass das herabfließende Wasservolumen auf den Scheitelpunkt des Winkels δ zuläuft. Hier gilt auch δ = χ - 270°. Der planare Zentralabschnitt ist optional und kann zugunsten eines geneigten Seitenabschnitts 6b auch verringert oder weggelassen werden. Der Kegelstumpf des Kopfabschnitts des Heizblocks wird dann zu einem Kegel.

Um zu verhindern, dass ein aus dem Dampfauslasskanal 31 zurücklaufendes Kondensatwasser in zeitlich unregelmäßiger Weise und unvorhersehbaren Volumina auf die Heizfläche trifft, weist der Bodenabschnitt des Dampfauslasskanals 31 ein sich parallel der Achse A erstreckendes Wasserrückführungselement 16 auf. Dieses ist vorliegend mittels einer Feder 17 federgelagert positioniert, um Fertigungstoleranzen auszugleichen. Das Wasserrückführungselement 16 kontaktiert die Heizfläche 6b physikalisch und thermisch, und wird so ebenfalls erwärmt. Dadurch wird ein regelmäßigerer Wasserrückfluss erzielt, und ein Leidenfrost-Effekt kann vermieden werden. Je steiler die Neigung des Dampfauslasskanals 31 gemäß dem Winkel β ist, desto gleichmäßiger ist die Wasserrückführung.

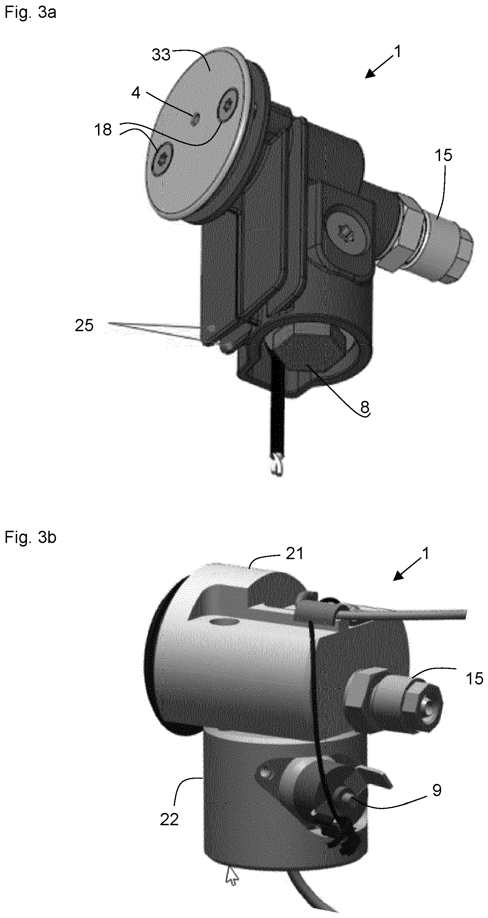

Fig. 3a zeigt die Verdampfervorrichtung 1, welche parallel zur Seitenwand 102 ausrichtbare Plattenelemente 25 des Bauteils 21/21' zur thermischen Abschirmung zwischen dem Heizblock 7 und der Seitenwand 102 beinhaltet. Gezeigt sind auch die Schrauben 25, mit denen das Verbindungselement 30 mit dem Bauteil 21/21' verschraubt wird.

Fig. 3b zeigt die Verdampfervorrichtung 1, mit erstem Bauteil 21 und zweitem Bauteil 22, sowie dem Temperatursensor 9.

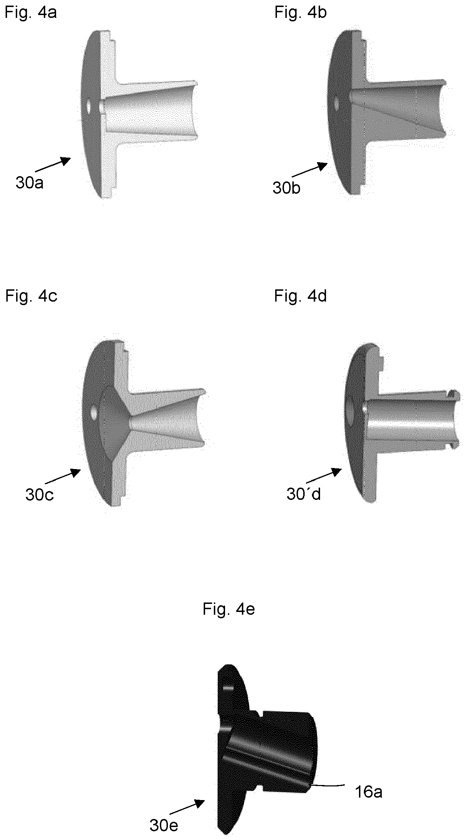

Fig. 4a bis 4e zeigen jeweils ein Ausführungsbeispiel des den Dampfauslasskanal 31 aufweisenden Verbindungselements 30, das zur Verbindung der Verdampfervorrichtung 1 gemäß Fig. 1a bis 3b mit der Seitenwand 102 einer Inkubationskammer 101 verwendbar ist.

In Fig. 4a bis 4e sind verschiedene Varianten des Dampfauslasskanals 31 gezeigt. In Untersuchungen hatte sich gezeigt, dass die Geometrie des Dampfauslasskanals sowohl das "Spuckverhalten" durch die Dampfaustrittsöffnung als auch das Regelverhalten beeinflussen kann. Die Formgebung des Verbindungselementes 30 wurde darauf ausgerichtet, dass möglichst kein Kondensat, das sich im Inneren bildet, in Richtung der Einlassbohrung läuft, da diese Tropfen durch den Überdruck im Inneren des Verdampfers nach außen geschleudert werden können. Zudem sollten sich nie größere Mengen Kondensat ansammeln, die sich dann schwallartig auf den Heizblock 7 ergießen und dann wiederum zum "Spucken" oder zu einem Überschießen der rH-Konzentration führen können. Die Variante aus Fig. 4b zeigte sich hinsichtlich des Spuckens als brauchbar, wenn auch relativ gesehen am wenigsten. Dies gilt abgeschwächt auch für den Dampfauslasskanals 31 aus Fig. 4c. Die Variante aus Fig. 4a zeigte hingegen ein gutes Verhalten, was insbesondere durch die leicht nach oben versetzte Bohrung der Dampfaustrittsöffnung 4 erreicht wurde. Zudem ist die Scheidewand 33 zur Inkubatorkammer sehr dünn ausgeführt, was das Risiko des Verstopfens der Bohrung 4 weiter reduziert. Die Fig 4d bündelt verschiedene wirksame Maßnahmen. Hier wurde zudem so geformt, dass die Innenkontur so angeschrägt ist, dass das Kondensatwasser auch oben weg von der Öffnung 4 in Richtung des Heizblocks läuft. Grundsätzlich sollte der Schrägenwinkel so steil wie möglich sein. Aufgrund der Bauraumrandbedingungen wurden hier 4° erreicht. Fig. 4e zeigt das Verbindungselement aus Fig. 2a. Es weist im Bodenabschnitt des Dampfkanals 31 eine längsverlaufende Aussparung zur Aufnahme des Stiftelements 16 auf.

Fig. 5a zeigt einen Inkubator 100 mit Verdampfervorrichtung 1. Das Wasser der Wasserpumpe 5 wird aus einem Wasserreservoir 50 bezogen, das oberhalb der Pumpeneinrichtung 5, hier auf der Gehäuseoberseite des Inkubators 100 angeordnet ist.

Fig. 5b zeigt ein System 200 aus einem Stapel von Verdampfervorrichtungen 1, die mit einem gemeinsamen Wasserreservoir 50 verbunden sind. Das Wasserreservoir 50 ist oberhalb jeder Pumpeneinrichtung 5, hier auf der Gehäuseoberseite des obersten Inkubators 100 angeordnet. Das Wasserreservoir 50 ist mit der Wasserfördereinrichtung 5 jeder der Verdampfervorrichtungen 1 verbunden. Das System 200 ist insbesondere so eingerichtet, dass im Betrieb mehrerer dieser Inkubatoren Wasser aus diesem einen Wasserreservoir 50 von allen Verdampfervorrichtungen 2 dieser Inkubatoren verdampft wird.

Fig. 5c zeigt das als Inkubator gebildete Laborgerät, an dessen Inkubatorkammerwand 102 die Verdampfervorrichtung 1 der Fig. 2 montiert ist. Die Inkubatorkammer 101 ist durch die Türe 111 verschlossen. Das Gehäuse des Laborgeräts ist größtenteils nicht gezeigt.

## Patentansprüche

1. Verdampfervorrichtung (1) zur Befeuchtung der Inkubatoratmosphäre eines Inkubators zur Inkubation lebender Zellkulturen, aufweisend
eine Verdampferkammer (2), die eine Wassereintrittsöffnung (3) zur Zuführung von flüssigem Wasser in die Verdampferkammer und eine mit dem Innenraum einer Inkubatorkammer des Inkubators verbindbare Dampfaustrittsöffnung (4) aufweist,
eine Wasserfördereinrichtung (5), mit der Wasser zu der Wassereintrittsöffnung beförderbar ist,
eine Heizfläche (6), die in der Verdampferkammer angeordnet ist und mit der ein mit der Heizfläche in Kontakt kommendes Wasser verdampfbar ist,
**dadurch gekennzeichnet, dass**
die Verdampfervorrichtung (1) eine Wasserführungseinrichtung (10) aufweist, durch die das von der Wassereintrittsöffnung (3) in die Verdampferkammer eingetretene Wasser bis zu der Heizfläche (6) an der Wasserführungseinrichtung (10) entlang fließend führbar ist.

2. Verdampfervorrichtung gemäß Anspruch 1, wobei die Wasserführungseinrichtung (10) eine Wasserführungsfläche (11) aufweist, die zwischen der Wassereintrittsöffnung und der Heizfläche (6) angeordnet ist, insbesondere um einen gravitationsbedingten Wasserfluss entlang der Wasserführungsfläche (11) zu ermöglichen.

3. Verdampfervorrichtung gemäß Anspruch 2, wobei die Verdampferkammer einen Innenwandabschnitt aufweist, der diese Wasserführungsfläche aufweist.

4. Verdampfervorrichtung gemäß einem der vorangehenden Ansprüche, wobei die Wasserführungseinrichtung so ausgebildet ist, dass das durch die Wassereintrittsöffnung austretende Wasser auf der Wasserführungsfläche fließt, insbesondere ohne dass ein freier Wassertropfen auf die Heizfläche fällt.

5. Verdampfervorrichtung gemäß einem der vorangehenden Ansprüche, wobei die Verdampfervorrichtung eine Wasserrückführungseinrichtung aufweist, durch die ein im Bereich zwischen der Dampfaustrittsöffnung und der Heizfläche auftretendes Kondenswasser bis zu der Heizfläche an der Wasserrückführungseinrichtung entlang fließend rückführbar ist.

6. Verdampfervorrichtung gemäß Anspruch 5, wobei die Wasserrückführungseinrichtung in thermischem Kontakt mit der Heizfläche steht.

7. Verdampfervorrichtung gemäß Anspruch 5 oder 6, wobei die Verdampfervorrichtung einen Dampfauslasskanal aufweist, der sich zwischen der Verdampferkammer und der Dampfaustrittsöffnung erstreckt, wobei die Wasserrückführungseinrichtung eine Wasserrückführungsfläche aufweist, die in einer Innenwand des Dampfauslasskanals ausgebildet ist und die sich zwischen der Dampfaustrittsöffnung und der Heizfläche erstreckt.

8. Verdampfervorrichtung gemäß einem der vorangehenden Ansprüche 5 bis 7, wobei die Wasserrückführungseinrichtung ein metallisches Wasserführungselement aufweist, dass sich zwischen der Dampfaustrittsöffnung und der Heizfläche erstreckt.

9. Verdampfervorrichtung gemäß einem der vorangehenden Ansprüche, wobei die Verdampfervorrichtung einen Dampfauslasskanal aufweist, der sich zumindest teilweise entlang einer Längsachse erstreckt, wobei die Dampfaustrittsöffnung einen kleineren Öffnungsquerschnitt aufweist als ein senkrecht zur Längsachse gemessener Querschnitt des Dampfauslasskanals.

10. Verdampfervorrichtung gemäß Anspruch 9, wobei die Dampfaustrittsöffnung azentrisch zu der Längsachse angeordnet ist, wobei die Dampfaustrittsöffnung oberhalb der einen Boden bildenden Heizfläche und oberhalb der zentralen Längsachse angeordnet ist, wenn die Verdampfervorrichtung im bestimmungsgemäßen Gebrauch an einer vertikalen Seitenwand einer Inkubatorkammer montiert ist.

11. Verdampfervorrichtung gemäß einem der vorangehenden Ansprüche, wobei die Wasserfördereinrichtung durch mindestens eine der folgenden technischen Daten gekennzeichnet ist:
a) das Wasserfördervolumen beträgt zwischen 1 µl und 50 µl, vorzugsweise zwischen 5 µl und 20 µl, vorzugsweise zwischen 10 µl und 20 µl, vorzugsweise zwischen 7 µl und 15 µl, vorzugsweise zwischen 9 µl und 11 µl;
b) die minimale Wasserförderrate beträgt zwischen 0.5 µl/h und 100 µl/h, vorzugsweise zwischen 0.5 µl/h und 20 µl/h, vorzugsweise zwischen 0.5 µl/h und 10 µl/h.

12. Verdampfervorrichtung gemäß einem der vorangehenden Ansprüche, die eine elektronische Steuereinrichtung aufweist, die dazu eingerichtet ist, die Wasserfördereinrichtung und die Heizfläche zur Regelung der Luftfeuchte in einer mit der Dampfaustrittsöffnung verbindbaren Inkubatorkammer zu betreiben.

13. Verdampfervorrichtung gemäß einem der vorangehenden Ansprüche, die einen Wasserzuführungskanal aufweist, der in die Wassereintrittsöffnung mündet, wobei ein Öffnungsquerschnitt der Wassereintrittsöffnung kleiner ist als ein Öffnungsquerschnitt des Wasserzuführungskanals.

14. Laborgerät zur Inkubation von Proben in einer Inkubationskammer, insbesondere Inkubator zur Inkubation lebender Zellkulturen, das eine Verdampfervorrichtung gemäß einem der vorangehenden Ansprüche aufweist.

15. Verfahren zum Erzeugen eines Wasserdampfes zur Befeuchtung der Atmosphäre in einer Inkubationskammer eines Laborgeräts, insbesondere eines Inkubators zur Inkubation lebender Zellkulturen, mittels einer eine Heizfläche aufweisenden Verdampfervorrichtung, insbesondere der Verdampfervorrichtung gemäß einem der Ansprüche 1 bis 13, wobei Wasser mittels einer Wasserführungseinrichtung an der Wasserführungseinrichtung entlang fließend bis zu der Heizfläche geführt wird, die das Wasser verdampft.
